# EUROPEAN PATENT APPLICATION

(11) **EP 2 187 202 A1**
(43) Date of publication of application: **19.05.2010**
(21) Application number: 08831153.5
(22) Date of filing: 27.08.2008
(51) Int. Cl.: G01N 27/12

(54) **SEMICONDUCTOR GAS SENSOR AND METHOD FOR MANUFACTURING THE SAME**

(30) Priority: 11.09.2007 JP 2007234807
(71) Applicant: The Ritsumeikan Trust, Kyoto-shi, Kyoto 604-8520 (JP); Horiba, Ltd., Kyoto-shi, Kyoto 601-8510 (JP)
(72) Inventor: TAMAKI, Jun, Kusatsu-shi Shiga 525-8577 (JP); NAKATA, Yoshiaki, Kyoto-shi Kyoto 601-8510 (JP); YAMAGISHI, Yutaka, Kyoto-shi Kyoto 601-8510 (JP)
(74) Representative: Hoffmann, Jörg Peter
(86) International application number: PCT/JP2008/065320
(87) International publication number: WO 2009/034843

(57) **Abstract**

This invention provides a semiconductor type gas sensor that can considerably increase the detection sensitivity to low-concentration gases, and can increase the response-recovery speed to achieve a conspicuous improvement in the overall performance, as well as a manufacturing method thereof.

This invention is a semiconductor type gas sensor including a semiconductor substrate 1 having a hollow portion 1a in a central part, an insulating film 2 of a diaphragm structure disposed on this substrate 1 to form to cover the hollow portion 1a, a heater 4 formed on this insulating film 2, a resistance-measuring electrode 6, and a gas-sensitive film 7 formed on the resistance-measuring electrode 6, **characterized in that** the gas-sensitive film 7 is made of monoclinic tungsten oxide containing a hexagonal tungsten oxide crystal.

## Description

### FIELD OF THE ART

The present invention relates to a semiconductor type gas sensor which is one kind of an environment monitoring sensor and is used, for example, for measurement of a nitrogen oxide (NOₓ) such as NO₂ which is one of air pollution components, as well as to a manufacturing method thereof. More particularly, the present invention relates to a semiconductor type gas sensor including a semiconductor substrate having a hollow portion in a central part, an insulating film of a diaphragm structure disposed on this substrate to form to cover the hollow portion, a heater formed on this insulating film, a resistance-measuring electrode, and a gas-sensitive film formed on the resistance-measuring electrode, as well as to a manufacturing method thereof.

### BACKGROUND ART

As a semiconductor type gas sensor such as an NO₂ gas sensor, performance of detecting low-concentration NO₂ of 0.01 ppm level at a sufficient sensitivity is demanded. As a sensor that meets such a demand for high-sensitivity performance, a sensor is conventionally known which is constructed in such a manner that a gas-sensitive film made of a monoclinic tungsten oxide (WO₃) crystal of a disk-shaped crystal powder is formed on a resistance-measuring electrode by dropping a tungstic acid (H₂WO₄) suspension liquid on the resistance-measuring electrode and sintering the product after drying, and NO₂ is measured by utilizing a property such that the resistivity of the monoclinic WO₃ crystal changes in accordance with the NO₂ gas concentration (for example, see Patent Documents 1 and 2).

Patent Document 1: Japanese Patent Application Laid-open (JP-A) No. 2007-64908
Patent Document 2: Japanese Patent Application Laid-open (JP-A) No. 6-102224

### DISCLOSURE OF THE INVENTION

### PROBLEMS TO BE SOLVED BY THE INVENTION

However, in a semiconductor type gas sensor conventionally known, the gas-sensitive film is formed only from a monoclinic WO₃ crystal, so that the detection sensitivity to low-concentration NO₂ is low, and the response-recovery characteristics are not good. In particular, it takes a long period of time for the recovery time, raising a problem in that the performance is insufficient to use the sensor for measurement of air pollution components. This point will be made clear also in the experiment example to be described later.

The present invention has been made in view of the above-described circumstances, and an object thereof is to provide a semiconductor type gas sensor that can considerably increase the detection sensitivity to low-concentration gases, and can increase the response-recovery speed to achieve a conspicuous improvement in the overall performance, as well as a manufacturing method thereof.

### MEANS FOR SOLVING THE PROBLEMS

A semiconductor type gas sensor according to the present invention that has been devised in order to achieve the aforementioned object is a semiconductor type gas sensor including a semiconductor substrate having a hollow portion in a central part, an insulating film of a diaphragm structure disposed on this substrate to expand to intercept the hollow portion, a heater formed on this insulating film, a resistance-measuring electrode, and a gas-sensitive film formed on the resistance-measuring electrode, **characterized in that** the gas-sensitive film is made of monoclinic tungsten oxide ( hereafter referred to as monoclinic WO₃) containing a hexagonal tungsten oxide crystal (hereafter referred to as a hexagonal WO₃ crystal) (claim 1).
Here, the gas-sensitive film is preferably formed by sintering a monoclinic tungsten oxide suspension liquid containing a hexagonal tungsten oxide crystal (hereafter referred to as a monoclinic WO₃ suspension liquid) on the resistance-measuring electrode (claim 2).

Also, a method of manufacturing a semiconductor type gas sensor according to the present invention that has been devised in order to achieve the same object as described above is a method of manufacturing a semiconductor type gas sensor including a semiconductor substrate having a hollow portion in a central part, an insulating film of a diaphragm structure disposed on this substrate to form to cover the hollow portion, a heater formed on this insulating film, a resistance-measuring electrode, and a gas-sensitive film formed on the resistance-measuring electrode, characterized by taking out a tungstic acid suspension liquid (hereafter referred to as an H₂WO₄ suspension liquid) by repeating suction filtration and water-washing processes for plural times after aging a precipitate obtained by adding an aqueous solution of (NH₄)₁₀W₁₂O₄₁·5H₂O into HNO₃ of 3 N to 6 N that is kept at a constant temperature, adding ion exchange water and a cationic surfactant to this H₂WO₄ suspension liquid that has been taken out, and dispersing the suspension liquid by stirring, preparing a monoclinic WO₃ suspension liquid containing a hexagonal WO₃ crystal by performing a hydrothermal treatment on this H₂WO₄ suspension liquid containing the surfactant at a temperature exceeding 140°C and below 160°C for 6 to 12 hours, dropping this monoclinic WO₃ suspension liquid containing a hexagonal WO₃ crystal on the resistance-measuring electrode, and forming a glass-sensitive film on the resistance-measuring electrode by sintering the suspension liquid at 300 to 400°C for 2 to 3 hours after drying (claim 6).

### EFFECTS OF THE INVENTION

According to the present invention having a construction as described above, since the gas-sensitive film formed on the resistance-measuring electrode contains a hexagonal WO₃ crystal whose resistivity changes extremely greatly depending on the gas concentration, the detection sensitivity to a low-concentration gas of ppb level can be outstandingly increased, and also the response speed and the recovery speed from gas exposure can be increased, thereby producing an effect such that the sensor can be used in a sufficiently effective manner in terms of performance also for measurement of air pollution components such as NO₂. Here, this point also will be made clear in the experiment example to be described later.

In the semiconductor type gas sensor according to the present invention, the monoclinic WO₃ suspension liquid containing the hexagonal WO₃ crystal is preferably one that has been synthesized by adding ion exchange water and a cationic surfactant to an H₂WO₄ suspension liquid and performing a thermal treatment on the resultant at a temperature exceeding 140°C and below 160°C for 6 to 12 hours, as recited in claim 3. More preferably, the monoclinic WO₃ suspension liquid containing the hexagonal WO₃ crystal is one that has been synthesized by adding ion exchange water and a cationic surfactant to an H₂WO₄ suspension liquid, adjusting the pH value to exceed 0.5 and below 2.5, and performing a hydrothermal treatment on this pH-adjusted resultant at a temperature exceeding 140°C and below 160°C for 6 to 12 hours, as recited in claim 4. By adopting such a synthesis condition, the rate of production of the hexagonal WO₃ crystal can be increased, whereby the detection sensitivity to a low-concentration gas can be further increased, and also the response speed and the recovery speed can be further increased.

Also, the gas-sensitive film in the semiconductor type gas sensor according to the present invention is preferably one that has been formed by dropping the monoclinic WO₃ suspension liquid containing the hexagonal WO₃ crystal on the resistance-measuring electrode, and sintering the suspension liquid at 300 to 400°C for 2 to 3 hours after drying, as recited in claim 5. By adopting such a sintering condition, a highly sensitive gas-sensitive film can be fixedly formed at a predetermined site on the resistance-measuring electrode with certainty and at a low cost under smaller electric power consumption. Here, the sintering may be carried out by using a high-temperature furnace or by energizing the heater that the gas sensor itself includes for heating.

### BEST MODES FOR CARRYING OUT THE INVENTION

Hereafter, embodiments of the present invention will be described with reference to the drawings.

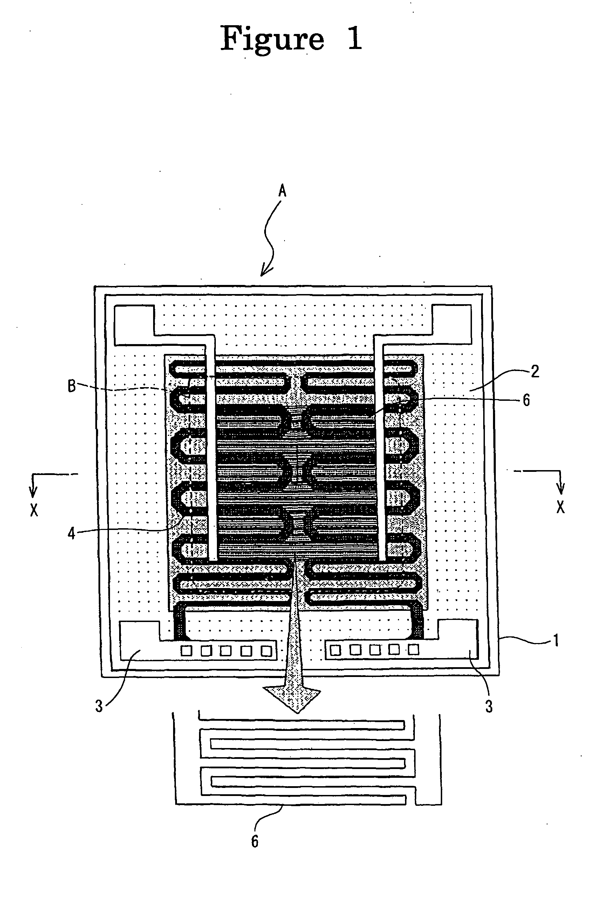

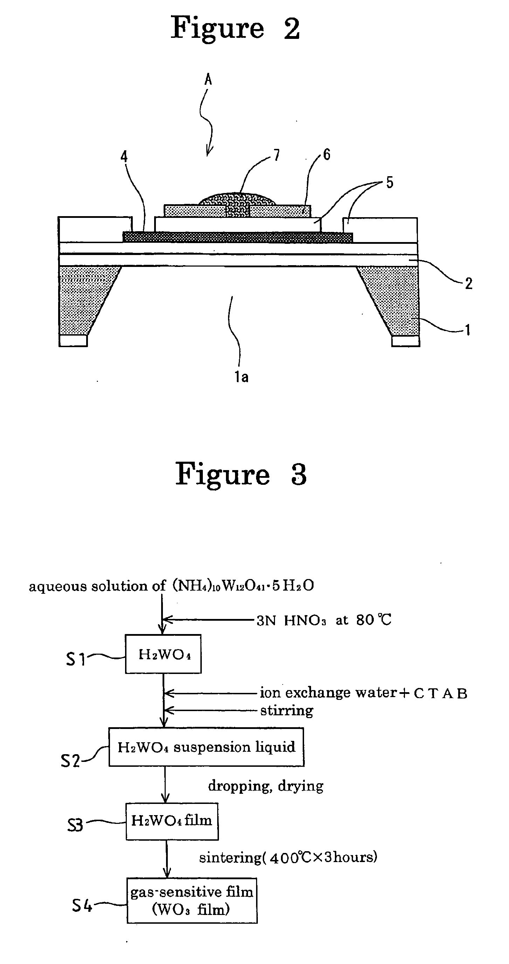

Fig. 1 is a plan view of a thin film type NO₂ sensor A which is one example of a semiconductor gas sensor according to the present invention. Fig. 2 is a longitudinal cross-sectional view along the line X-X of Fig. 1.

This thin film type NO₂ sensor A is provided with a silicon (Si) substrate 1 having a hollow portion 1a of a rectangular shape in a plan view at a central part, an SiO₂ insulating film 2 of a rectangular diaphragm structure formed to cover the hollow portion 1a on the Si substrate 1 by inserting this Si substrate 1 into, for example, an oxidation furnace and oxidizing both the front and back surfaces thereof to a thickness of 2000 ± 500 Å, a heater 4 formed on this insulating film 2 and receiving application of a constant voltage by electrodes 3, 3 for energization, a resistance-measuring electrode 6 formed on an insulating film 5 made by etching necessary sites after forming a non-silicate glass (NSG) film having a thickness of 4000 ± 500 Å on this heater 4 by the CVD method or the like, and a gas-sensitive film 7 formed on this resistance-measuring electrode 6.

The heater 4 is formed in a pattern shape whose density at the peripheral part is the largest and whose density gradually decreases according as it approaches the central part, formed by etching in a predetermined double-zigzag pattern shape by the photolithography method after forming a metal film made of a hardly-oxidizable high-melting point material such as platinum (Pt) by the sputtering method or the like to a thickness of 3000 ± 500 Å in a range corresponding to the generally whole region of the rectangular hollow portion 1a in the Si substrate 1 on the insulating film 2. In more detail, the heater 4 is formed in a double-zigzag pattern shape such that the heater line width and the heater line interval (pitch) are both the minimum at both of the side portions of the rectangular insulating film 2 that oppose each other, and both the heater line width and the pitch increase gradually according as they approach the central part. By this, it is constructed in such a manner that, when the heater 4 is energized for heating via the electrodes 3, 3 for energization, the temperature of the whole of the rectangular region B surrounded by the dotted line on the insulating film 2 can be raised to a uniform temperature in relation to the Joule heat. Here, as the heater 4, tantalum (Ta) or tungsten (W) may be used besides the aforesaid platinum.

As clearly shown by taking out an essential part at the lower part of Fig. 1, the aforesaid resistance-measuring electrode 6 is formed in a comb-shaped pattern that occupies almost the whole region within the uniform temperature range B by the heater 4. In more detail, after forming a metal film of gold (Au) or the like to a thickness of 5000 ± 500 Å by the sputtering method or the like on the insulating film 5, the metal film is etched into a predetermined comb-shaped pattern by the photolithography technique, thereby to form the resistance-measuring electrode 6 having a line interval of 5 µm and a line width of 5 µm.

Also, the aforesaid gas-sensitive film 7 is formed to occupy the most part of the comb-shaped pattern on the resistance-measuring electrode 6. Hereafter, steps of forming this gas-sensitive film 7 will be described in detail with reference to Fig. 3.

First, 5.012 g of (NH₄)₁₀W₁₂O₄₁·5H₂O is dissolved into 200 ml of water to prepare an aqueous solution of (NH₄)₁₀W₁₂O₄₁·5H₂O of 8 mmol. In the meantime, 44.8 ml of 30% HNO₃ is diluted with water to form 100 ml of a solution, so as to prepare HNO₃ of 3 N. Next, while keeping the HNO₃ of 3 N at 80°C, the aqueous solution of (NH₄)₁₀W₁₂O₄₁·5H₂O of 8 mmol is added little by little by a dropper, so as to obtain a dark yellow precipitate. After this precipitate is aged in a dark place for 24 hours, suction filtration and water-washing processes are repeated for plural times (about three times), so as to take out the precipitate H₂WO₄ (step S1).

To this H₂WO₄ that has been taken out, 50 ml of ion exchange water is added, and 0.0164g {one-fold of critical micelle concentration (cmc)} of a cationic surfactant (cetyltrimethylammonium bromide [CH₃(CH₂)₁₅N(CH₃)₃]Br: CTAB) is added to this and, after the pH value is adjusted to exceed 0.5 and below 2.5, the mixture is stirred in a dark place with use of a magnetic stirrer for two weeks or more, so as to prepare an H₂WO₄ suspension liquid containing the surfactant. This H₂WO₄ suspension liquid containing the surfactant is sufficiently dispersed and put into a pressure-resistant container made of stainless steel not illustrated in the drawings, and a hydrothermal treatment is carried out in an oven that is kept at a temperature exceeding 140°C and below 160°C, for example, at 150°C as a preferable example, for 6 to 12 hours, for example, for 10 hours as a preferable example. After the treatment, the resultant is left to stand and cooled to room temperature, thereby to prepare a monoclinic WO₃ suspension liquid containing a hexagonal WO₃ crystal (step S2).

The monoclinic WO₃ suspension liquid containing a hexagonal WO₃ crystal prepared by a synthesis method as described above is dropped onto the resistance-measuring electrode 6. After the resultant is dried to form a WO₃ film (step S3), the WO₃ film is sintered in a high-temperature furnace at 300 to 400°C for 2 to 3 hours, for example, at 400°C for 3 hours as a preferable example, thereby to form a predetermined gas-sensitive film 7 on the resistance-measuring electrode 6 (step S4).

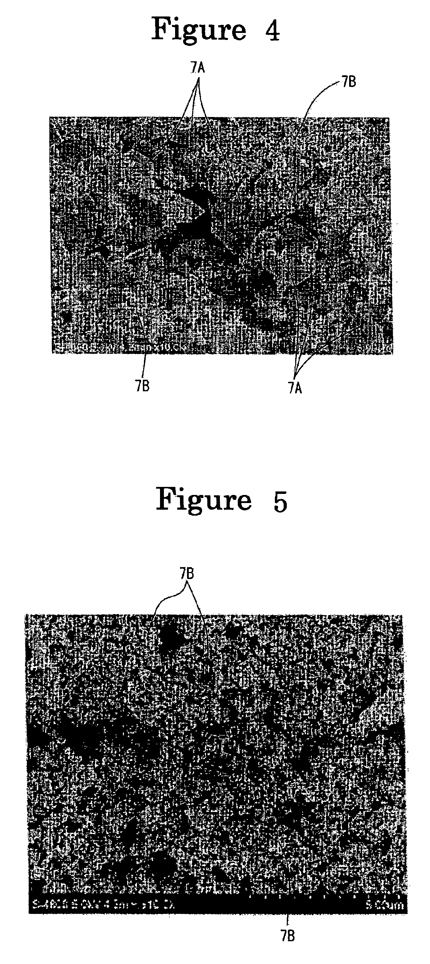

The WO₃ powder corresponding to the example of the present invention that had been produced under the hydrothermal treatment condition of 150°C for 10 hours from the H₂WO₄ suspension liquid containing the surfactant (CTAB) prepared by a synthesis method as described above, and the WO₃ powder corresponding to the comparative example that had been produced under the hydrothermal treatment condition of 150°C for 10 hours from the H₂WO₄ suspension liquid without containing the surfactant (CTAB) prepared by a synthesis method similar to the above were each subjected to SEM imaging, whereby SEM images as shown in Figs. 4 and 5 were obtained. As will be clear from the SEM images shown in Figs. 4 and 5, in the WO₃ powder corresponding to the example of the present invention, hexagonal WO₃ crystals 7A of a hexagonal plate shape crystal with one side being about 1.5 µm and monoclinic WO₃ crystals 7B of a cuboid shape crystal powder with one side being about 50 to 100 nm were mixedly present. In contrast, the WO₃ powder corresponding to the comparative example was all made of monoclinic WO₃ crystals 7B of a cuboid shape crystal powder with one side being about 50 to 100 nm, so that hexagonal WO₃ crystals 7A of a hexagonal plate shape crystal were not present.

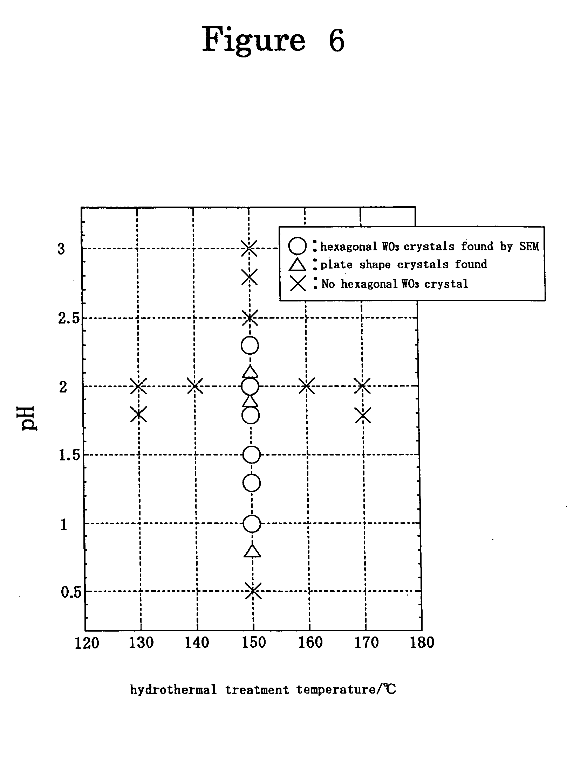

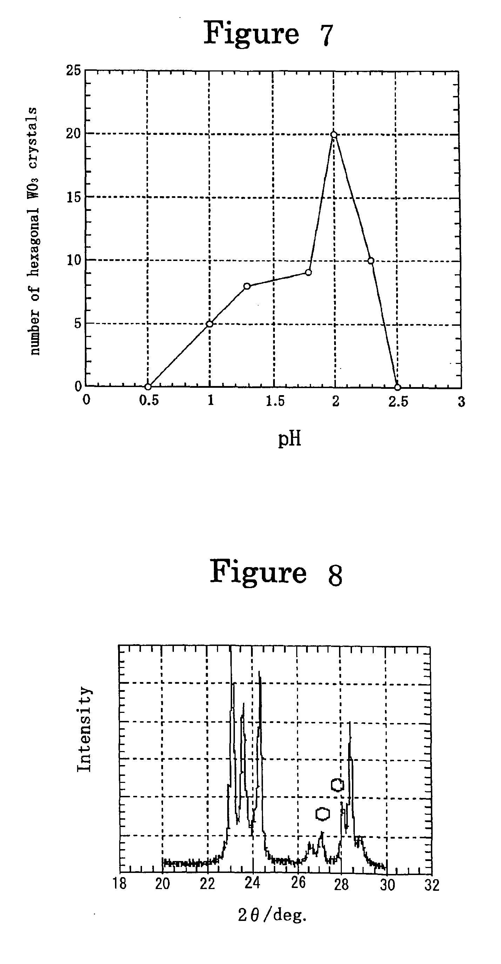

Also, when a relationship between the hydrothermal treatment temperature and the pH value was analyzed on the basis of each of the above-described SEM images, results such as shown in Fig. 6 were obtained. Further, when a relationship between the pH value of the H₂WO₄ suspension liquid and the number of the hexagonal WO₃ crystals was examined on the basis of Fig. 6, results such as shown in Fig. 7 were obtained. As will be clear from these results shown in Figs. 6 and 7, the hexagonal WO₃ crystal of a hexagonal plate-shaped crystal is produced in a case in which a hydrothermal treatment is carried out at a temperature exceeding 140°C and below 160°C, and the hydrothermal treatment is most preferably carried out at 150°C. Also, it has been confirmed that the hexagonal WO₃ crystals are produced when the pH value of the H₂WO₄ suspension liquid is adjusted to be 0.5 or more and 2.5 or below, and that the hexagonal WO₃ crystals are produced in the largest number when the pH value is within a range from 1.7 to 2.4.

Also, when X-ray diffraction was carried out on the WO₃ powder corresponding to the example of the present invention that had been produced under the above-described synthesis condition and the hydrothermal treatment condition of 150°C for 10 hours, an XRD diagram such as shown in Fig. 8 was obtained. In this Fig. 8, the hexagonal marks at 27.1° and at 28.2° represent hexagonal WO₃ crystals, and the other peaks represent monoclinic WO₃ crystals.

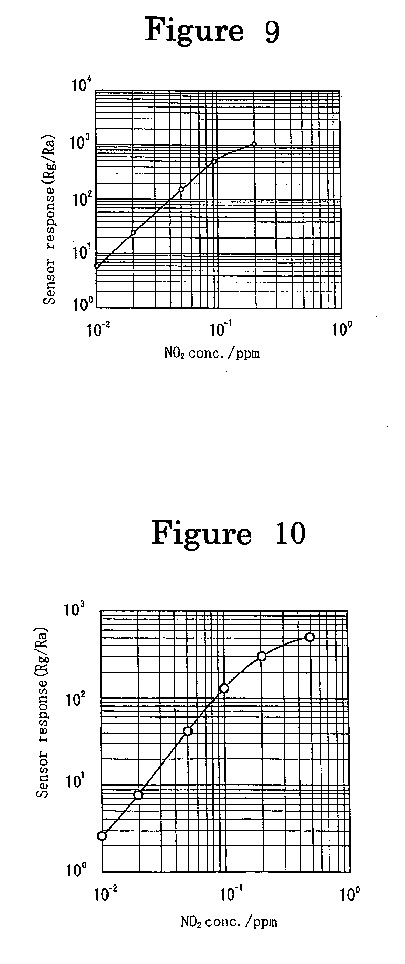

Next, the present inventors carried out an experiment on the concentration dependency of the NO₂ sensitivity of the thin film type NO₂ sensor A of the embodiment of the present invention in which the gas-sensitive film 7 had been formed by dropping the monoclinic WO₃ suspension liquid containing the hexagonal WO₃ crystal produced under the synthesis condition and the hydrothermal treatment condition described above on the resistance-measuring electrode 6 and sintering the resultant in a high-temperature furnace at 400°C for 3 hours after drying, and the thin film type NO₂ sensor of the comparative example in which the gas-sensitive film 7 had been formed by dropping the H₂WO₄ suspension liquid on the resistance-measuring electrode 6 and sintering the resultant in a high-temperature furnace at 400°C for 3 hours after drying. As a result thereof, sensitivity curves as shown in Fig. 9 (embodiment of the present invention) and in Fig. 10 (comparative example) were obtained.

As will be clear from the above-described experiment results, it has been confirmed that the thin film type NO₂ sensor A of the embodiment of the present invention exhibits an extremely high detection sensitivity within a range of 0.01 to 0.2 ppm such that the sensor sensitivity S (Rg/Ra) is 6 when the NO₂ concentration is 0.01 ppm and S = 1000 when the NO₂ concentration is 0.2 ppm, as shown in Fig. 9, so that even low-concentration NO₂ of 0.01 ppm can be detected at a sufficiently high sensitivity. On the other hand, it has been found out that the thin film type NO₂ sensor of the comparative example has a low sensitivity as a whole to low-concentration NO₂ with NO₂ of 0.01 ppm being the detection limit, as shown in Fig. 10, and cannot be used in terms of performance for the measurement of air pollution components in which NO₂ of a concentration lower than that is present.

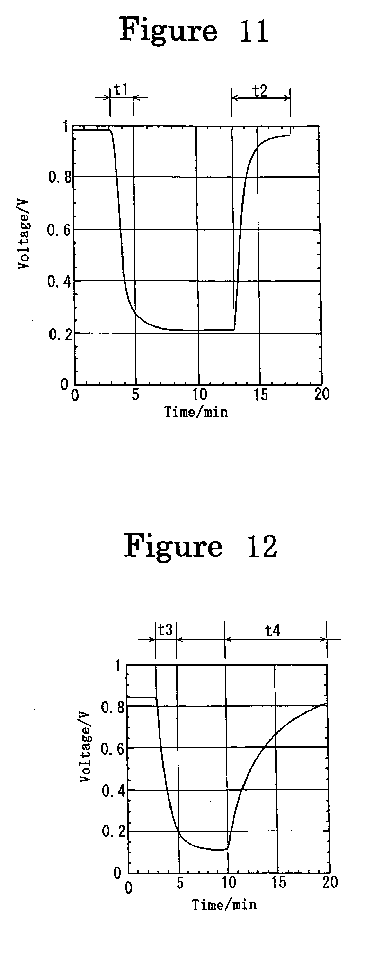

Also, an experiment was carried out to determine a response curve to 0.05 ppm (50 ppb) NO₂ of the thin film type NO₂ sensor A of the embodiment of the present invention and the thin film type NO₂ sensor of the above-described comparative example. As a result of this, response curves such as shown in Fig. 11 (embodiment of the present invention) and in Fig. 12 (comparative example) were obtained.

As will be clear from the above-described experiment results, it has been confirmed that, in the thin film type NO₂ sensor A of the embodiment of the present invention, the 90% response time t1 is 1.5 minutes, and the 90% recovery time t2 is 1.5 minutes, so that both the response speed and the recovery speed are high, and the sensor A can be sufficiently applied to continuous measurement of air pollution components having a low concentration. On the other hand, it has been found out that, in the thin film type NO₂ sensor of the comparative example, the 82% response time t3 is 1.5 minutes, and the 80% recovery time t4 is 10 minutes, so that both the response speed and the recovery speed are low, and the sensor cannot be practically used for measurement of air pollution components in which continuous measurement is carried out.

Here, in the above-described embodiment, the heater 4 is shown to be formed in a double-zigzag pattern shape such that the density of the heater 4 is the maximum in the peripheral part of the rectangular range B and the density decreases gradually according as it approaches the central part in order to widen the uniform temperature range. However, the heater 4 may be formed in a double-zigzag pattern shape such that the density of the whole region is equal by making the heater line width and the heater line interval (pitch) be identical both in the peripheral part and in the central part.

Also, a description has been made on a case in which, in forming a gas-sensitive film 7 by dropping a monoclinic WO₃ suspension liquid containing a hexagonal WO₃ crystal on the resistance-measuring electrode 6 and sintering the resultant after drying, the gas-sensitive film 7 is formed by sintering the suspension liquid in a high-temperature furnace at 400°C for 3 hours. However, the gas-sensitive film 7 can be formed by sintering caused by energization and heating of the heater 4 itself of the thin film type NO₂ sensor A. In this case, the electric power consumption for sintering can be reduced, whereby reduction of production costs of the sensor can be achieved.

### INDUSTRIAL APPLICABILITY

With the semiconductor type gas sensor according to the present invention, the detection sensitivity to a low-concentration gas of ppb level can be outstandingly increased, and the response speed and the recovery speed from gas exposure can be increased, so that it can be used for measurement of an air pollution component such as NO₂ in a sufficiently effective manner.

### BRIEF DESCRIPTION OF THE DRAWINGS

[FIG. 1] Fig. 1 is a plan view of a thin film type NO₂ sensor of an embodiment which is one example of a semiconductor gas sensor according to the present invention.
[FIG. 2] Fig. 2 is a longitudinal cross-sectional view along the line X-X of Fig. 1.
[FIG. 3] Fig. 3 is a view showing a step of forming a gas-sensitive film in the thin film type NO₂ sensor of the embodiment of the present invention.
[FIG. 4] Fig. 4 is a view illustrating an SEM image of a WO₃ powder produced by performing a hydrothermal treatment at 150°C for 10 hours on a H₂WO₄ suspension liquid containing a surfactant which is used in the embodiment of the present invention.
[FIG. 5] Fig. 5 is a view illustrating an SEM image of a WO₃ powder produced by performing a hydrothermal treatment at 150°C for 10 hours on a H₂WO₄ suspension liquid without containing a surfactant which is used as a comparative example.
[FIG. 6] Fig. 6 is a chart showing a result of analyzing a relationship between the hydrothermal temperature and the pH on the basis of the SEM image of the WO₃ powder.
[FIG. 7] Fig. 7 is a chart showing a result of examining a relationship between the pH of the H₂WO₄ suspension liquid and the number of crystals of the hexagonal WO₃ crystal on the basis of Fig. 6.
[FIG. 8] Fig. 8 is an XRD diagram obtained by X-ray diffraction of the WO₃ powder produced by performing a hydrothermal treatment at 150°C for 10 hours on a H₂WO₄ suspension liquid containing a surfactant which is used in the embodiment of the present invention.
[FIG. 9] Fig. 9 is a concentration - sensitivity curve graph showing a result of experiment on the concentration dependency of the NO₂ sensitivity of the thin film type NO₂ sensor of the embodiment of the present invention.
[FIG. 10] Fig. 10 is a concentration - sensitivity curve graph showing a result of experiment on the concentration dependency of the NO₂ sensitivity of the thin film type NO₂ sensor of the comparative example.
[FIG. 11] Fig. 11 is a response curve graph showing a result of performing an experiment for determining a response curve on 0.05 ppm NO₂ of the thin film type NO₂ sensor of the embodiment of the present invention.
[FIG. 12] Fig. 12 is a response curve graph showing a result of performing an experiment for determining a response curve on 0.05 ppm NO₂ of the thin film type NO₂ sensor of the comparative example.

### DESCRIPTION OF THE SYMBOLS

- A: thin film type NO₂ sensor (one example of semiconductor gas sensor)
- 1: Si substrate
- 1a: hollow portion
- ·: insulating film
- 4: heater
- 6: resistance-measuring electrode
- 7: gas-sensitive film
- 7A: hexagonal WO₃ crystal
- 7B: monoclinic WO₃ crystal

## Claims

1. A semiconductor type gas sensor including a semiconductor substrate having a hollow portion in a central part, an insulating film of a diaphragm structure disposed on this substrate to form to cover the hollow portion, a heater formed on this insulating film, a resistance-measuring electrode, and a gas-sensitive film formed on the resistance-measuring electrode, **characterized in that** the gas-sensitive film is made of monoclinic tungsten oxide containing a hexagonal tungsten oxide crystal.

2. The semiconductor type gas sensor according to claim 1, wherein the gas-sensitive film is formed by sintering a monoclinic tungsten oxide suspension liquid containing a hexagonal tungsten oxide crystal on the resistance-measuring electrode.

3. The semiconductor type gas sensor according to claim 2, wherein the monoclinic tungsten oxide suspension liquid containing the hexagonal tungsten oxide crystal is one that has been synthesized by adding ion exchange water and a cationic surfactant to a tungstic acid suspension liquid and performing a thermal treatment on the resultant at a temperature exceeding 140°C and below 160°C for 6 to 12 hours.

4. The semiconductor type gas sensor according to claim 2, wherein the monoclinic tungsten oxide suspension liquid containing the hexagonal tungsten oxide crystal is one that has been synthesized by adding ion exchange water and a cationic surfactant to a tungstic acid suspension liquid, adjusting the pH value to exceed 0.5 and below 2.5, and performing a hydrothermal treatment on this pH-adjusted resultant at a temperature exceeding 140°C and below 160°C for 6 to 12 hours.

5. The semiconductor type gas sensor according to either of claims 1 through 4 wherein the gas-sensitive film is one that has been formed by dropping the monoclinic tungsten oxide suspension liquid containing the hexagonal tungsten oxide crystal on the resistance-measuring electrode, and sintering the suspension liquid at 300 to 400°C for 2 to 3 hours after drying.

6. A method of manufacturing a semiconductor type gas sensor including a semiconductor substrate having a hollow portion in a central part, an insulating film of a diaphragm structure disposed on this substrate to form to cover the hollow portion, a heater formed on this insulating film, a resistance-measuring electrode, and a gas-sensitive film formed on the resistance-measuring electrode,
**characterized by** taking out a tungstic acid suspension liquid by repeating suction filtration and water-washing processes for plural times after aging a precipitate obtained by adding an aqueous solution of (NH₄)₁₀W₁₂O₄·5H₂O into HNO₃ of 3 N to 6 N that is kept at a constant temperature, adding ion exchange water and a cationic surfactant to this tungstic acid suspension liquid that has been taken out, and dispersing the suspension liquid by stirring, preparing a monoclinic tungsten oxide suspension liquid containing a hexagonal tungsten oxide crystal by performing a hydrothermal treatment on this tungstic acid suspension liquid containing the surfactant at a temperature exceeding 140°C and below 160°C for 6 to 12 hours, dropping this monoclinic tungsten oxide suspension liquid containing a hexagonal tungsten oxide crystal on the resistance-measuring electrode, and forming a glass-sensitive film on the resistance-measuring electrode by sintering the suspension liquid at 300 to 400°C for 2 to 3 hours after drying.

7. The method of manufacturing a semiconductor type gas sensor according to claim 6, wherein the monoclinic tungsten oxide suspension liquid containing the hexagonal tungsten oxide crystal is one that has been synthesized by adding ion exchange water and a cationic surfactant to a tungstic acid suspension liquid, adjusting the pH value to exceed 0.5 and below 2.5, and performing a hydrothermal treatment on this pH-adjusted resultant at a temperature exceeding 140°C and below 160°C for 6 to 12 hours.
